# EUROPÄISCHE PATENTANMELDUNG

(11) **EP 0 677 247 A1**
(43) Veröffentlichungstag der Anmeldung: **18.10.1995**
(21) Anmeldenummer: 95104949.3
(22) Anmeldetag: 03.04.1995
(51) Int. Cl.: A01N 63/00

(54) **Insektizide Mischungen**

(30) Priorität: 14.04.1994 DE 4412834
(71) Anmelder: BAYER AG, 51368 Leverkusen (DE)
(72) Erfinder: Schnorbach, Hans-Jürgen, Dr., D-40789 Monheim (DE)

(57) **Zusammenfassung**

Die vorliegende Erfindung betrifft insektizide Mischungen aus Bacillus spp. Präparaten und Agonisten oder Antagonisten der nicotinergen Acetylcholinrezeptoren von Insekten und ihre Verwendung.

## Beschreibung

Die vorliegende Erfindung betrifft insektizide Mischungen aus Bacillus spp. Präparaten und Agonisten oder Antagonisten der nicotinergen Acetylcholinrezeptoren von Insekten und ihre Verwendung.

Bacillus thuringiensis Präparate und ihre insektizide Verwendung sind bekannt (z.B. aus (Chemical Abstracts Zitate) CA 71/122 392; CA 80/129 289 g; CA 86/1701 d; CA 118/100 539 f; sowie DE-OS 2 146 165; EP-OS 63 949; EP-OS 93 062; EP-OS 142 924; EP-PS 153 166; PCT-OS WO 86/01 536; EP-OS 178 151; EP-OS 200 344; EP-OS 216 481; DE-OS 3 541 893; PCT-OS WO 88/06 631; EP-OS 308 199 PCT-OS WO 88 08877; PCT-OS WO 91/09 133; EP-OS 500 311 x.).

Agonisten oder Antagonisten der nicotinergen Acetylcholinrezeptoren von Insekten sind bekannt, z.B. aus den folgenden Publikationen:
Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 91/4965; Französische Anmeldung Nr. 2 611 114; Brasilianische Anmeldung Nr. 88 03 621.

Auf die in diesen Publikationen beschriebenen Methoden, Verfahren, Formeln und Definitionen sowie auf die darin beschriebenen einzelnen Präparationen und Verbindungen wird hiermit ausdrücklich Bezug genommen.

Mischungen von Bacillus thuringiensis Präparaten mit Insektiziden verschiedener Wirkstoffklassen sind bekannt (Chemical Abstracts Zitat CA 117/247 152 n; 113/226 446 p; 107/54 201 n; JP-Pat. 1 576 352; DE-OS 2 250 085).

Es sind jedoch noch keine Mischungen bekannt geworden von Bacillus spp. Präparaten mit Agonisten oder Antagonisten der nicotinergen Acetylcholinrezeptoren von Insekten.

Gegenstand der vorliegenden Erfindung sind:
1. Insektizide Mittel, gekennzeichnet durch einen Gehalt einer Mischung aus Bacillus spp. Präparaten, Bacillus spp. Endotoxine enthaltenden Präparaten sowie transkonjugante und rekombinante Bacillus spp. Präparate und Agonisten oder Antagonisten der nicotinergen Acetylcholinrezeptoren von Insekten.
2. Verbesserung der insektiziden Wirkung von Bacillus spp. Endotoxine enthaltenden Präparaten sowie transkonjuganten und rekombinanten Bacillus spp. Präparaten, dadurch gekennzeichnet, daß man ihnen Agonisten oder Antagonisten der nicotinergen Acetylcholinrezeptoren von Insekten zusetzt.
3. Verwendung von Mischungen aus Bacillus spp. Präparaten, Bacillus spp. Endotoxine enthaltenden Präparaten sowie transkonjuganten und rekombinanten Bacillus spp. Präparaten und Agonisten oder Antagonisten der nicotinergen Acetylcholinrezeptoren bei Insekten zur Bekämpfung von Insekten, anderen Arthropoden und Nematoden.
4. Verfahren zur Bekämpfung von Insekten, anderen Arthropoden und Nematoden, dadurch gekennzeichnet, daß man eine Mischung aus Bacillus spp. Präparaten, Bacillus spp. Endotoxine enthaltenden Präparaten sowie transkonjuganten und rekombinanten Bacillus spp. Präparaten und Agonisten oder Antagonisten der nicotinergen Acetylcholinrezeptoren bei Insekten auf Insekten und/oder ihren Lebensraum einwirken läßt.
5. Verfahren zur Herstellung von insektiziden Mitteln, dadurch gekennzeichnet, daß man Bacillus spp. Präparate, Bacillus spp. Endotoxine enthaltende Präparate sowie transkonjugante und rekombinante Bacillus spp. Präparate und Agonisten oder Antagonisten der nicotinergen Acetylcholinrezeptoren von Insekten mit Streck- und/oder Verdünnungsmitteln und oberflächenaktiven Stoffen vermischt.

Es war überraschend, daß sich die Wirkung von Bacillus spp. Präparaten durch Kombination mit Agonisten oder Antagonisten der nicotinergen Acetylcholinrezeptoren von Insekten steigern läßt.

Bacillus spp. Präparate sind insbesondere Bacillus thuringiensis Präparate, deren Endotoxine und rekombinant hergestellte Stämme.

Agonisten oder Antagonisten der nicotinergen Acetylcholinrezeptoren von Insekten werden z.T. unter dem Begriff Nitromethylene und damit verwandte Verbindungen zusammengefaßt.

Diese Verbindungen lassen sich bevorzugt durch die allgemeine Formel (I) wiedergeben 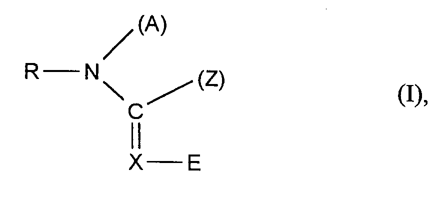
in welcher
- R: für Wasserstoff, gegebenenfalls substituierte Reste der Gruppe Acyl, Alkyl, Aryl, Aralkyl, Heteroaryl oder Heteroarylalkyl steht;
- A: für eine monofunktionelle Gruppe aus der Reihe Wasserstoff, Acyl, Alkyl, Aryl steht oder für eine bifunktionelle Gruppe steht, die mit dem Rest Z verknüpft ist;
- E: für einen elektronenziehenden Rest steht;
- X: für die Reste -CH= oder =N- steht, wobei der Rest -CH= anstelle eines H-Atoms mit dem Rest Z verknüpft sein kann;
- Z: für eine monofunktionelle Gruppe aus der Reihe Alkyl, -O-R, -S-R, 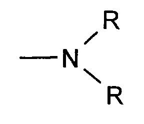 steht oder für eine bifunktionelle Gruppe steht, die mit dem Rest A oder dem Rest X verknüpft ist.

Besonders bevorzugt sind Verbindungen der Formel I, in welcher die Reste folgende Bedeutung haben:
- R: steht für Wasserstoff sowie für gegebenenfalls substituierte Reste aus der Reihe Acyl, Alkyl, Aryl, Aralkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.
Als Alkyl seien genannt C₁₋₁₀-Alkyl, insbesondere C₁₋₄-Alkyl, im einzelnen Methyl, Ethyl, i-Propyl, sec.- oder t.-Butyl, die ihrerseits substituiert sein können.
Als Aryl seien genannt Phenyl, Naphthyl, insbesondere Phenyl.
Als Aralkyl seien genannt Phenylmethyl, Phenethyl.
Als Heteroaryl seien genannt Heteroaryl mit bis zu 10 Ringatomen und N, O, S insbesondere N als Heteroatomen. Im einzelnen seien genannt Thiophenyl, Furyl, Thiazolyl, Imidazolyl, Pyridyl, Benzthiazolyl.
Als Heteroarylalkyl seien genannt Heteroarylmethyl, Heteroarylethyl mit bis zu 6 Ringatomen und N, O, S, insbesondere N als Heteroatomen.
Als Substituenten seien beispielhaft und vorzugsweise aufgeführt:
Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo (-SO₃H); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.
- A: steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.
- A und Z: können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Hexahydro-1,3,5-triazin, Morpholin genannt, die gegebenenfalls bevorzugt durch Methyl substituiert sein können.
- E: steht für einen elektronentziehenden Rest, wobei insbesondere NO₂, CN, Halogenalkylcarbonyl wie 1,5-Halogen-C₁₋₄-carbonyl, insbesondere COCF₃ genannt seien.
- X: steht für -CH= oder -N=
- Z: steht für gegebenenfalls substituierte Reste Alkyl, -OR, -SR, -NRR, wobei R und die Substituenten bevorzugt die oben angegebene Bedeutung haben.
- Z: kann außer dem oben genannten Ring gemeinsam mit dem Atom, an welches es gebunden ist und dem Rest 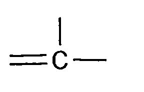 an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II) und (III) genannt: 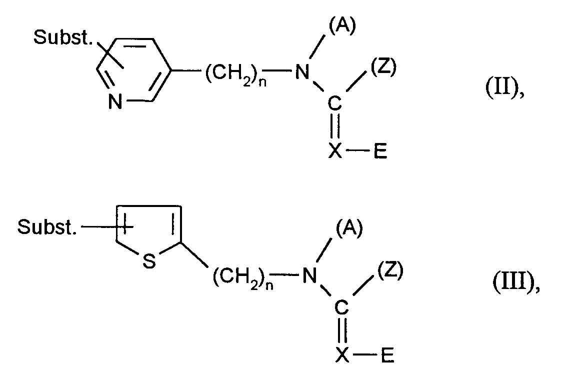
in welchen
- n: für 1 oder 2 steht,
- Subst.: für einen der oben aufgeführten Substituenten, insbesonders für Halogen, ganz besondere für Chlor, steht,
- A, Z, X und E: die oben angegebenen Bedeutungen haben,
Im einzelnen seien folgende Verbindungen genannt: 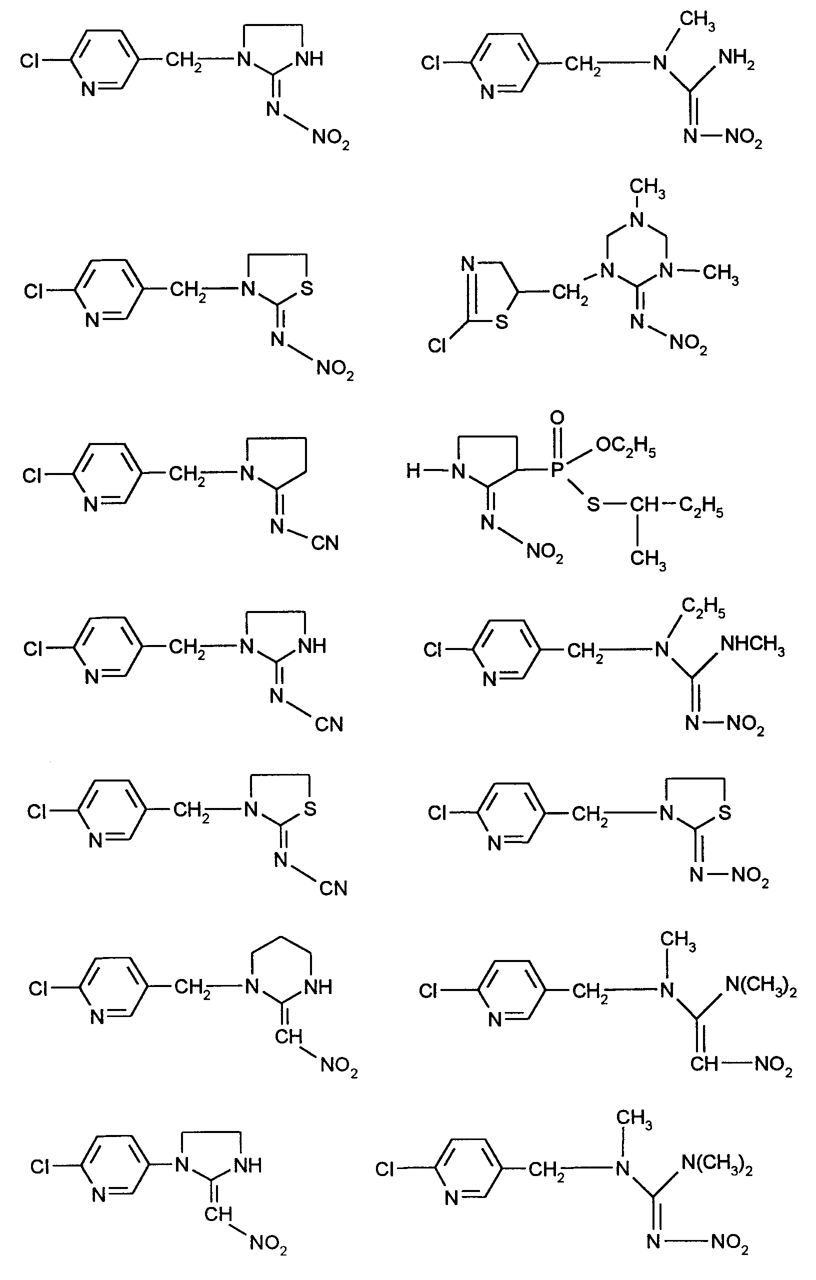 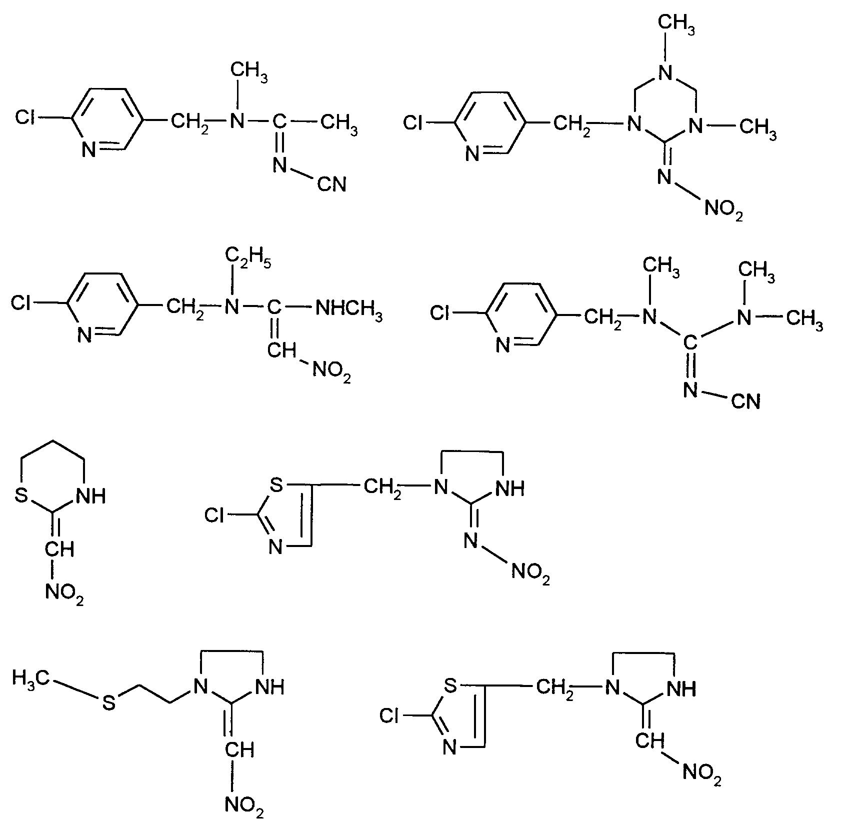
Die erfindungsgemäßen Mischungen eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porecellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pedculus humanus corporis, Haematopinus spp., Linognathus spp..

Aus der Ordnung der Mallophaga z.B. Trichodectes spp., Damalinea spp..

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp..

Aus der Ordnung der Homoptera z.B. Phylloxera vastatrix, Pemphigus spp., Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Heliothis spp.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus pp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tanaus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Lactrodectus mactans.

Bevorzugt seien genannt kauend beißende Insekten, z.B. aus den Ordnungen Coleoptera oder Lepidoptera.

Die erfindungsgemäßen Mischungen können in ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffmischung, vorzugsweise zwischen 0,5 und 90 %.

Die erfindungsgemäßen Mischungen können noch weitere Wirkstoffe, wie Insektizide, Lockstoffe, Sterilantien, Akarizide, Nematizide, Fungizide, wachstumsregulierende Stoffe oder Herbizide enthalten. Zu den Insektiziden zählen beispielsweise Phosphorsäureester, Carbamate, Carbonsäureester, chlorierte Kohlenwasserstoffe, Phenylharnstoffe u.a.

Die erfindungsgemäßen Mischungen können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Agonsiten oder Antagonisten der nicotinergen Acetylcholinrezeptoren von Insekten in den Anwendungsformen kann von 0,0001 bis 1 Gew.-% Wirkstoff, vorzugsweise zwischen 0,0016 und 0,1 Gew.-% liegen. Der Wirkstoffgehalt im Fall der Bacillus thuringiensis Präparate der Anwendungsformen kann von 10²-10¹² I.U. pro 100 ml, bevorzugt 10⁴-10⁷ I.U. pro 100 ml, variieren.

Die Anwendung geschieht in einer den Anwendungsformen angepaßten üblichen Weise durch Spritzen, Vernebeln, Stäuben, Sprühen, Gießen.

Bei der Anwendung gegen Hygiene- und Vorratsschädlinge zeichnen sich die chemischen Wirkstoffe durch eine hervorragende Residualwirkung auf Holz und Ton sowie durch eine gute Alkalistabilität auf gekälkten Unterlagen aus.

In den folgenden Beispielen wird als Vertreter eines Agonisten oder Antagonisten der nicotinergen Acetylcholinrezeptoren von Insekten die Verbindung mit dem common name Imidacloprid (1-(2-Chlor-5-pyridinyl-methyl)-2-nitroiminoimidazolidin) und als Vertreter der Gattung Bacillus thuringiensis, vorzugsweise die Stämme B.t.var. kurstaki, -aizawai, -tenebrionis, -israelensis eingesetzt.

Imidacloprid wurde in Form des Handelspräparates ^{®}Confidor eingesetzt.

Kombinationen von Imidacloprid mit unterschiedlichen Konzentrationen an Bacillus thuringiensis wurden in Laborversuchen im Vergleich zu den Einzelkomponenten biologisch geprüft. Um den gegenseitigen Einfluß der Wirkstoffe auf ihre insektizide Wirkung feststellen zu können, wurden niedrige Wirkstoffkonzentrationen im Bereich des Wirkungsabbruchs gewählt, die alleine keine volle Wirkung bringen. Als Versuchstiere dienen Larven von Phaedon cochleariae, Spodoptera frugiperda bzw. Soja, Plutellaxylostellas, Heliothis armigera. Die Larven befanden sich im L2-Stadium. Blätter von Wirsingkohl (Brassica oleracea var. Sabauda) wurden in die Prüflösungen getaucht und nach dem Abtrocknen mit dem Schädling infiziert. Die Auswertung erfolgte 3 Tage nach der Infektion. Neben der insektiziden Wirkung (Mortaliät) wurde auch die Schutzwirkung (Fraßgrad) bonitiert. Die Versuche wurden jeweils zweimal wiederholt.

### Versuchsergebnisse in tabellarischer Darstellung

### Beispiel 1

Ergebnisse der Versuche mit Phaedon cochleariae und der Kombination Imidacloprid / Bacillus thuringiensis var. kurstaki (^{®}Dipel 2x, Fa. Abbott)

| Präparat/Kombination | Wirkung nach 3 Tagen Mortalität (%) |
|---|---|
| Imidacloprid (0,0008 %) | 2 |
| B. t. var. kurstaki (5,0 x 10E5 I.U./100 ml) | 2 |
| B. t. var. kurstaki (2,5 x 10E6 I.U./100 ml) | 0 |
| Imidacloprid (0,0008%) und B. t. var. kurstaki (5,0 x 10E5 I.U./100 ml) | 19 |
| Imidacloprid (0,0008%) und B. t. var. kurstaki (2,5 x 10E6 I.U./100 ml) | 65 |
| Kontrolle | 0 |

### Beispiel 2

Ergebnisse der Versuche mit Phaedon cochleariae und der Kombination Imidacloprid / Bacillus thuringiensis var. aizawai (^{®}Xentari, Fa. Abbott)

| Präparat/Kombination | Wirkung nach 3 Tagen Mortalität (%) |
|---|---|
| Imidacloprid (0,0008 %) | 2 |
| B. t. var. aizawai (2,0 x 10E6 I.U./100 ml) | 0 |
| B. t. var. aizawai (1,0 x 10E7 I.U./100 ml) | 0 |
| Imidacloprid (0,0008%) und B. t. var. aizawai (2,0 x 10E6 I.U./100 ml) | 20 |
| Imidacloprid (0,0008%) und B. t. var. aizawai (1,0 x 10E7 I.U./100 ml) | 50 |
| Kontrolle | 0 |

### Beispiel 3

Ergebnisse der Versuche mit Phaedon cochleariae und der Kombination Imidacloprid / Bacillus thuringiensis var. tenebrionis (^{®}Novodor 2 %, Fa. Neudorff)

| Präparat/Kombination | Wirkung nach 3 Tagen Mortalität (%) |
|---|---|
| Imidacloprid (0,0008 %) | 3 |
| Imidacloprid (0,004 %) | 40 |
| B. t. var. tenebrionis (0,00012 %) | 3 |
| B. t. var. Tenebrionis (0,0006 %) | 23 |
| Imidacloprid (0,0008 %) und B. t. var. tenebrionis (0,00012 %) | 33 |
| Imidacloprid (0,0008 %) und B. t. var. tenebrionis (0,0006 %) | 47 |
| Imidacloprid (0,004 %) und B. t. var. tenebrionis (0,00012 %) | 100 |
| Imidacloprid (0,004 %) und B. t. var. tenebrionis (0,0006 %) | 100 |
| Kontrolle | 0 |

### Beispiel 4

Ergebnisse der Versuche mit Spodoptera frugiperda und der Kombination Imidacloprid / Bacillus thuringiensis var. tenebrionis (^{®}Novodor 2 %, Fa. Neudorff)

| Präparat/Kombination | Wirkung nach 3 Tagen Mortalität (%) |
|---|---|
| Imidacloprid (0,004 %) | 50 |
| B. t. var. tenebrionis (0,0006 %) | 0 |
| B. t. var. tenebrionis (0,003 %) | 3 |
| Imidacloprid (0,004 %) und B. t. var. tenebrionis (0,0006 %) | 80 |
| Imidacloprid (0,004 %) und B. t. var. tenebrionis (0,003 %) | 90 |
| Kontrolle | 7 |

### Beispiel 5

Ergebnisse der Versuche mit Spodoptera frugiperda und der Kombination Imidacloprid / Bacillus thuringiensis var. kurstaki (^{®}Dipel 2x, Fa. Abbott)

| Präparat/Kombination | Wirkung nach 3 Tagen Mortalität (%) |
|---|---|
| Imidacloprid (0,0008 %) | 7 |
| B. t. var. kurstaki (2,0 x 10E6 I.U./100 ml) | 18 |
| Imidacloprid (0,0008%) und B. t. var. kurstaki (2,0 x 10E6 I.U./100 ml) | 51 |
| Kontrolle | 0 |

## Patentansprüche

1. Insektizide Mittel, gekennzeichnet durch einen Gehalt einer Mischung aus Bacillus spp. Präparaten, Bacillus spp. Endotoxine enthaltenden Präparaten sowie transkonjugante und rekombinante Bacillus spp. Präparate und Agonisten oder Antagonisten der nicotinergen Acetylcholinrezeptoren von Insekten.

2. Verbesserung der insektiziden Wirkung von Bacillus spp. Endotoxine enthaltenden Präparaten sowie transkonjuganten und rekombinanten Bacillus spp. Präparaten, dadurch gekennzeichnet, daß man ihnen Agonisten oder Antagonisten der nicotinergen Acetylcholinrezeptoren von Insekten zusetzt.

3. Verwendung von Mischungen aus Bacillus spp. Präparaten, Bacillus spp. Endotoxine enthaltenden Präparaten sowie transkonjuganten und rekombinanten Bacillus spp. Präparaten und Agonisten oder Antagonisten der nicotinergen Acetylcholinrezeptoren bei Insekten zur Bekämpfung von Insekten, anderen Arthropoden und Nematoden.

4. Verfahren zur Bekämpfung von Insekten, anderen Arthropoden und Nematoden, dadurch gekennzeichnet, daß man eine Mischung aus Bacillus spp. Präparaten, Bacillus spp. Endotoxine enthaltenden Präparaten sowie transkonjuganten und rekombinanten Bacillus spp. Präparaten und Agonisten oder Antagonisten der nicotinergen Acetylcholinrezeptoren bei Insekten auf Insekten und/oder ihren Lebensraum einwirken läßt.

5. Verfahren zur Herstellung von insektiziden Mitteln, dadurch gekennzeichnet, daß man Bacillus spp. Präparate, Bacillus spp. Endotoxine enthaltende Präparate sowie transkonjugante und rekombinante Bacillus spp. Präparate und Agonisten oder Antagonisten der nicotinergen Acetylcholinrezeptoren von Insekten mit Streck- und/oder Verdünnungsmitteln und oberflächenaktiven Stoffen vermischt.
